# EUROPEAN PATENT SPECIFICATION

(11) **EP 2 498 850 B1**
(45) Date of publication and mention of the grant of the patent: **08.01.2014**
(21) Application number: 10778883.8
(22) Date of filing: 15.11.2010
(51) Int. Cl.: A61M 16/04

(54) **TRACHEAL TUBE WITH TEMPERATURE SENSOR**
TRACHEALTUBUS MIT TEMPERATURSENSOR
CANULE TRACHÉALE COMPORTANT UN CAPTEUR DE TEMPÉRATURE

(30) Priority: 13.11.2009 DE 102009053067
(43) Date of publication of application: 19.09.2012
(73) Proprietor: Willy Rüsch GmbH, 71394 Kernen-Rommelshausen (DE)
(72) Inventor: SINGVOGEL, Armin, 71686 Remsbeck (DE); LIM, Li San, 34600 Taiping (Perka) (MY)
(74) Representative: Grünecker, Kinkeldey, Stockmair & Schwanhäusser
(86) International application number: PCT/EP2010/006940
(87) International publication number: WO 2011/057818

(56) References cited:
- WO-A2-2006/002364
- DE-U1- 9 416 465
- US-A- 4 046 139
- US-A- 5 038 777
- US-A- 5 295 489
- US-A1- 2003 041 863

## Description

The present invention refers to a tracheal tube for respiration of patients by using a breathing tube, which is at least partially insertable into the trachea, comprising a cuff arranged at the part insertable into the trachea around the breathing tube, and comprising a temperature sensor.

Tracheal tubes for respiration of patients suffering from insufficient or irregular respiration are known in the art in various forms and embodiments and serve for introducing respiratory gas into the lungs of a patient. For this purpose, the shaft of a tube is endotracheally or tracheostomatically inserted into the trachea of the patient. A cuff is provided at the distal end of the part of the breathing tube inserted into the trachea, said cuff ensuring the sealing between the breathing tube and the trachea. For this purpose the trachea sealing cuff is first of all filled by a gas as soon as the breathing tube is located at its destined position in the trachea. Usually, the cuff is filled with gas independent of the respiratory air, wherein the pressure when inflating the cuff is set to the lowest possible value in order to avoid traumata. The inflated cuff effects that the respiratory gas reaches the lower parts of the respiratory system to thereby ensure sufficient oxygen supply of the patient.

Patients, who are ventilated by means of a tracheal tube, must frequently also be monitored as to their general clinical state with respect to body temperature. The precise measurement of the body core temperature is an important aspect of clinical monitoring in the context of general anesthesia and other critical states. When administering blood transfusions and intravenous liquids an excessive temperature can lead to a significant rise of the body core temperature of the patient and it can increase the oxygen consumption of the body, which can cause hypoxia of the brain and other organs. Administering too cold intravenous solutions can, on the other hand, lead to hypothermia, which can cause a decrease of the oxygen content in the body. To monitor the body core temperature separate temperature probes are usually placed in various orifices of the body. Besides oral and anal temperature measurement, special temperature probes exist for the tympanic membrane, the nasopharynx, the esophagus, the lungs and the rectum.

The position for these temperature probes must be localized carefully and the probe itself must safely be fixed.

Caused by the frequent connection of monitoring the body core temperature of endotracheally or tracheostomatically ventilated patients, the combination of temperature sensors with tracheal tubes for the respiration of patients has long been known in the prior art.

DE 19543072 A1 describes for instance a tracheal tube comprising a temperature sensor for measuring the body core temperature. The temperature sensor is arranged at the distal end of the breathing tube below the cuff. Although this arrangement of the sensor neither affects the sealing nor the trachea itself, however, the temperature sensor is arranged at a significant distance to the trachea. Additionally, the proximity of the sensor to the outlet orifice for the respiratory gas influences the accuracy of temperature measurement.

US 4,046,139 on the other hand shows a temperature sensor, which substantially abuts a wall of the trachea. The sensor is either attached at the tip of the breathing tube or on the inner side of the trachea sealing cuff. In these embodiments in impreciseness of temperature measurement has also proven as having an adverse effect.

WO 2006/002364 discloses an endotracheal tube comprising an outer and an inner cuff and a temperature sensor which lies in the inner cuff. Thus, the present invention is based on the object of avoiding or improving the disadvantages of the embodiments of tracheal tubes with temperature sensors known from the prior art.

This object is solved by the generic tracheal tube with temperature sensor according to the invention in that the cuff has an outer balloon and an inner balloon and the temperature sensor is arranged between the outer balloon and the inner balloon and is attached on the outer side of the inner balloon.

Such a tracheal tube with temperature sensor according to the invention enables by the arrangement of the sensor between the outer balloon and the inner balloon of the trachea sealing cuff a favorable and uniform contact pressure of the temperature sensor with respect to the trachea and thereby also a favorable rest of the sensor on the trachea which is required for temperature measurement and that is only separated by the low wall thickness of the outer balloon. The temperature sensor is on the one hand protected by the outer balloon against corrosive or aggressive influences from the trachea, on the other hand the outer balloon reduces the risk of possible traumatic damage on the trachea caused by the temperature sensor. The integration of the temperature sensor between the outer balloon and the inner balloon of the cuff therefore enables a safe and precise measurement of the body core temperature. Compared to the tracheal tubes known in the prior art having dual or double cuffs, which are either arranged offset with respect to each other or stacked on one another on the breathing tube, the outer balloon and the inner balloon of the one cuff of the tracheal tube according to the invention only have a low distance to each other, which substantially serves for receiving the measuring head and the connection wires of the temperature sensor.

A purposeful embodiment provides that the temperature sensor is attached on the outer side of the inner balloon by means of an adhesive. Such an arrangement of the temperature sensor enables besides the simple attachment also a free and specific positioning of the temperature sensor in the cuff and thus also with respect to the trachea. Besides the attachment of the temperature sensor on the outer side of the inner balloon, an attachment on the inner side of the outer balloon is also possible. Furthermore, the temperature sensor can be embedded on the outer side of the inner balloon, whereby the temperature sensor projects less with respect to the outer side of the inner balloon and therefore also exerts a lower selective pressure via the outer balloon to the trachea.

A preferred modification allows that a protective layer is provided, and the protective layer covers the temperature sensor towards the outer balloon. The protective layer further reduces the selective pressure of the temperature sensor onto the trachea and thereby reduces the risk of traumatic damage in the trachea. The protective layer can be positioned independent of the attachment of the temperature sensor between the temperature sensor and the outer balloon. Particularly when the temperature sensor is attached on or in the inner balloon, the protective layer can also easily be attached on the outer side of the inner balloon above the temperature sensor. Furthermore, the protective layer or a further protective layer can cover the temperature sensor with respect to the inner balloon to avoid a perforation of the inner balloon by the measuring head of the temperature sensor or its terminals. It is also advantageous if the protective layer is a metal foil, preferably an aluminum foil. The design of the protective layer of a metal foil or aluminum foil improves the head conductance of the trachea and increases the surface of the trachea considered for the temperature measurement. The use of a protective metal layer increases the accuracy of the measurement of the body core temperature and reduces the risk of systematic measuring errors caused by positioning problems. It is advantageous for the manufacture of the tracheal tube according to the invention that the metal foil or aluminum foil is a self-adhesive foil.

The outer balloon and the inner balloon of the cuff of a tracheal tube according to the invention can usually be made of the same material. The use of the same materials reduces particularly in connection with the same configuration the expenditure in manufacturing. Conventional high-volume/low-pressure trachea sealing cuffs for a uniform and gentle contact of the envelope sealing face on the trachea are made of a flexible material. The diameter of the balloon is dimensioned so large that it already contacts the trachea at a very low inflation pressure of e.g. 15 millibar without revealing a significant flexible deformation. The entire wall thickness of a cuff composed of an outer balloon and an inner balloon is compared to conventional cuffs composed of one membrane only with a wall thickness of up to approx. 0.2 mm somewhat larger, however, it remains below the double value. The wall thicknesses of the outer balloon and of the inner balloon amount to approx. 0.1. to 0.15 mm, wherein the wall thickness of the inner balloon can be significantly lower caused by the additional protection of the outer balloon than the wall thickness of the outer balloon.

A further embodiment of the tracheal tube according to the invention provides that the inner balloon of the cuff is made of a more flexible material than the outer balloon. Caused by the use of differently flexible materials for the inner balloon and the outer balloon an improved contact pressure behavior of the temperature sensor to the outer balloon of the cuff and thus to the trachea of the patient can be reached. Furthermore, a bulge caused by the arrangement of the temperature sensor between the inner balloon and the outer balloon extends substantially towards the inside so that the trachea is substantially not stressed.

A favorable embodiment provides that the temperature sensor is a NTC sensor. An NTC resistance sensor or hot-carrier thermal resistor is well adapted for use in measuring the body core temperature. An NTC sensor conducts current better at high temperatures than at low temperatures so that a negative temperature coefficient (NTC) is produced. Caused by the limited application area the electrical resistance changes more upon changing temperatures than with other sensors, so that besides a sensitive and fast reaction of the NTC sensor a high precision is also possible. For the use in the tracheal tube according to the invention the measuring head of the NTC resistance sensor is preferably formed spherically or knob-shaped. With a measuring head diameter of approx. 1.5 mm, the risk of injury for the mucosa of the trachea but also the risk of a perforation of the outer balloon or the inner balloon of the cuff can be reduced. A suitable temperature sensor is for instance provided by the company Betatherm Sensors under the designation Betacurve, which is particularly adapted for use in medical application.

The connection wires of the temperature sensor can purposefully extend in the cuff up to the breathing tube between the inner balloon and the outer balloon. Such a positioning of the connection wires of the temperature sensor leading to the measuring head prevents by a passage of the connection wires through the inner balloon into the interior of the cuff and thus it also prevents the risk of possible defects and holes in the inner balloon. Defects and holes in the inner balloon lead to a deterioration of the contact pressure of the sensor or of the measuring head on the trachea, since part of the pressure exerted in the trachea sealing cuff is not output via the inner balloon but directly onto the outer balloon. By placing the connection wires between the inner balloon and the outer balloon of the cuff the corrosion protection reached by the respective positioning of the temperature sensor is further improved with respect to corrosive influences from the trachea and with respect to the medium used for pressure build-up in the cuff.

To reduce the load and traumatic damage, the connection wires of the temperature sensor at least in the part insertable into the trachea can be arranged within and/or integrated in the breathing tube. The part of the tube insertable into the trachea is the part of the breathing tube, which is inserted into the body or the trachea of the patient when using the tracheal tube for ventilating patients, usually 50 to 80 % of the length of the breathing tube. Occasionally, the part of the tube that is not inserted into the trachea is shortened in clinical use and the connection piece for connection with the respirator is inserted onto the shortened tube again so that the part of the breathing tube actually inserted into the trachea can also be significantly larger. In the part of the breathing tube insertable into the trachea the connection wires of the temperature sensor and ventilation cannulas for supplying the cuff are arranged integrated in and/or parallel to the breathing tube. Such a positioning of the connection wires and of the ventilating cannulas avoids the presence of mutually interfering lines and reduces the number of the lines to be inserted into the trachea so that both intubation itself as well as the presence of the tracheal tube in the trachea is less stressful for the patient and interfering influences are avoided.

The structure and the mode of operation of an embodiment of the present invention will now be explained in detail by means of the enclosed drawing.
Fig. 1 shows a tracheal tube according to the invention comprising a cuff arranged in the trachea,
Fig. 2 shows an enlarged view of the cuff of Fig. 1,
Fig. 3 shows the temperature sensor of Fig. 2, and
Fig. 4 shows an enlarged view of the cuff of Fig. 2 in a view rotated about 90°.

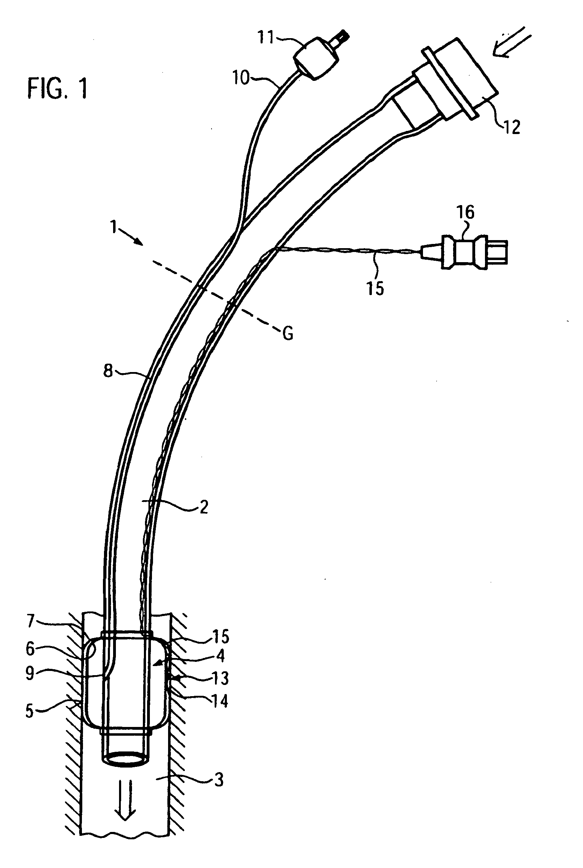

Fig. 1 shows a tracheal tube according to the invention for the endotracheal use in a side view. The tracheal tube 1 comprises a flexible breathing tube 2, which in a known manner is inserted into the trachea 3, and which is connected on its upper end with a pressure controlled respirator (now shown), which serves for the periodical inspiration of respiratory gas into a patient's lungs. The breathing tube 2 is provided on its lung-sided end inserted into the trachea 3 with a trachea sealing cuff 4. The barrel-shaped cuff 4 is made of a thin flexible material and rests with its outer envelope sealing surface 5 on the wall of the trachea 3 to ensure sealing between the breathing tube 2 and the trachea 3. Besides the barrel-shaped cuff 4 shown, a cylindrical or spherical design of the cuff 4 is also possible for the tracheal tube 1 according to the invention. The trachea sealing cuff 4 is composed of an inner balloon 6 and an outer balloon 7, wherein the outer balloon 7 completely encompasses the inner balloon 6. The inner balloon 6 and the outer balloon 7 are both connected on their front end sides with the breathing tube 2 in an air-tight manner. The outer balloon 7 is preferably made of a non-transparent material, whereas the inner balloon 6 is made of a material that is flexible compared to the material of the outer balloon 7, which contrary to the outer balloon 7 can also be transparent.

The cuff 4 is filled by means of a ventilation cannula 8 with a pressurized gas. The ventilation cannula 8 extends in the part of the breathing tube 2 insertable into the trachea 3, i.e. between the border G of the part of the breathing tube 2 insertable into the trachea and the cuff 4, within the wall or in the interior of the breathing tube 2 and opens with its opening 9 into the inner balloon 6 of the cuff. On the distal end 10 facing away from the cuff 4 the ventilation cannula 8 opens into a separate connecting piece 11, which serves for filling the trachea sealing cuff 4 and at the same time as a return valve for maintaining the inflation pressure in the cuff 4. The breathing tube 2 opens at the end not insertable into the trachea 3, i.e. the end facing away from the cuff 4, into a connecting piece 12, which is used for ventilating the patient by a usually pressure-controlled respirator.

A temperature sensor 13 is embedded between the inner balloon 6 and the outer balloon 7 of the cuff 4 in the area of the envelope seal surface 5 resting on the trachea 3. The measuring head 14 of the temperature sensor 13 is fixed in position via the inner balloon 6 or the outer balloon 7. The connecting wires 15 of the temperature sensor 13 connected with the measuring head 14 extend between the inner balloon 6 and the outer balloon 7 along the envelope sealing surface and the front end side of the cuff facing away from the lungs to the breathing tube 2 and in the area of the connection of the inner balloon 6 and the outer balloon with the breathing tube 2 through the breathing tube 2 into the interior of the breathing tube 2. In the interior of the breathing tube 2 the connecting wires 15 extend without or with an additional protection by a suitable sleeve or embedding into the wall of the breathing tube 2 up to an area of the breathing tube 2 above the border G of the part of the breathing tube 2 insertable into the trachea 3. Above the border G the connecting wires 15 are passed through the breathing tube 2 towards the outside and end in a plug 16, which can be connected to a respective device for monitoring the body core temperature.

Besides the embodiment of the tracheal tube 1 for endotracheal application shown in Fig. 1, a tracheal tube 1 according to the invention can be formed in the same way for a tracheostomatomatic application. The structure and the mode of operation is adapted according to the cuff 4 arranged at the lung-sided end of the breathing tube 2 with temperature sensors 13 according to the tracheal tube 1 for endotracheal applications shown in Fig. 1.

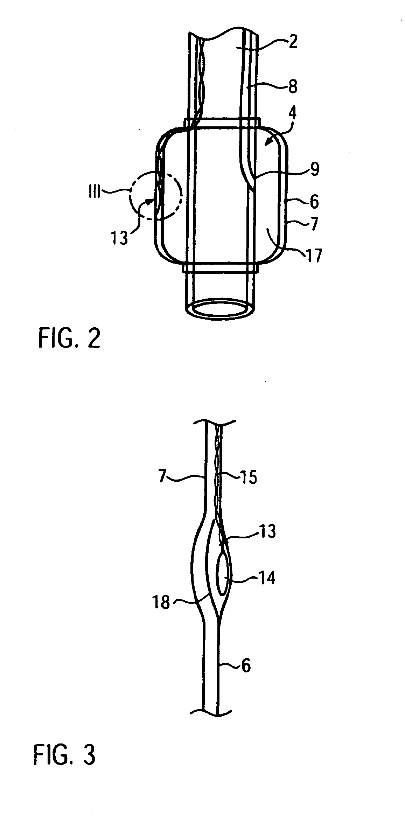

Fig. 2 shows the trachea sealing cuff 4 arranged at the lung-sided end of the breathing tube 2 in an enlarged view. Besides the barrel-shaped design of the cuff 4, the inner balloon 6 and the outer balloon 7 can also clearly be recognized in this view. The opening 9 of the ventilation cannula 8 opens through the wall of the breathing tube 2 into the annular pressure chamber 17 formed between the inner balloon 6 and the breathing tube 2. The temperature sensor 13 is arranged between the inner balloon 6 and the outer balloon 7 on the side of the cuff 4 facing away from the opening 9 of the ventilation cannula 8.

As can more clearly be seen in the enlarged view of the detail III of Fig. 2 in Fig. 3, the spherical or knob-shaped measuring head 14 of the temperature sensor 13 is attached on the outer side of the inner balloon 6, e.g. by means of an adhesive and/or a form-fit connection. The connecting wires 15 extend from the measuring head 14 between the inner balloon 6 and the outer balloon 7 up to the connection of the cuff 4 at the breathing tube 2. The measuring head 14 and the surrounding area are covered by a protective foil 18 made of aluminum. The measuring head is not only additionally or alternatively fixed on the outer side of the inner balloon, but the end of the connecting wires 15 as well as their connection of the measuring head 14 is also protected. The measuring head 14 of the temperature sensor 13 is embedded between the inner balloon 6 and the protective foil 18.

The outer balloon 7 is arranged on the outer side of the inner balloon 6 or above the protective foil 18 facing away from the pressure chamber 17, wherein the inner side of the outer balloon 7 substantially contacts the outer side of the inner balloon 6 and the outer side of the protective foil 18.

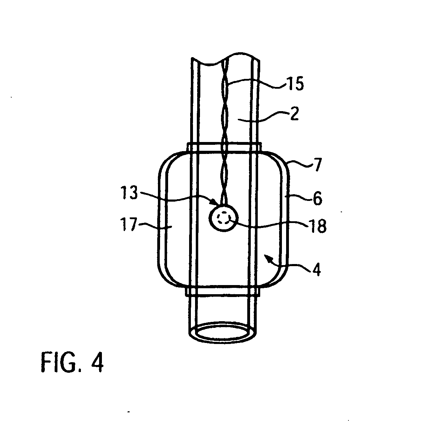

Fig. 4 shows an illustration of the enlarged view of the cuff 4 of Fig. 2 rotated by 90°. Again, the temperature sensor 13 is arranged between the inner balloon 6 and the outer balloon 7 of the cuff 4 and the connecting wires 15 extend between the inner balloon 6 and the outer balloon 7 to the breathing tube 2 and further within the breathing tube 2 up to above of the border G for the part of the breathing tube 2 insertable into the trachea. In this illustration the protective foil 18 covering the measuring head 14 and the connection of the connecting wires 15 to the measuring head can clearly be seen.

The mode of operation of the tracheal tube 1 with temperature sensor 13 will now be explained in detail.

After inserting the tracheal tube 1 into the trachea 3 of a patient, the pressure chamber 17 between the inner balloon 6 of the cuff 4 and the breathing tube 2 is filled by a gas via the connecting piece 11 and the ventilation cannula, and it is loaded by an inflation pressure, wherein the inflation pressure of the trachea sealing cuff 4 is to be limited to a possibly low value for the just sufficient sealing to minimize the traumatic stress caused by the contact pressure of the cuff against the walls of the trachea. When filling the pressure chamber 17 the inner balloon 6 expands and rests with its outer side on the inner side of the outer balloon 7. Since the outer balloon 7, as in conventional high-volume low-pressure cuffs has a diameter adapted to the size of the trachea 3, the outer side evenly and smoothly rests at the mucosa of the trachea also at low inflation pressure. Since the inner balloon 6 is also composed of a flexible material, the outer wall of the inner balloon evenly rests at the inner wall of the outer balloon 7. Thereby a favorable contact of the temperature sensor 13 covered by the protective foil 18 arranged on the outer wall of the inner balloon 6 is achieved. Irrespective of whether the inner balloon 6 is made of a more flexible or more elastic material than the outer balloon 7, the flexibility of the inner balloon 6 in connection with the low inflation pressure in the pressure chamber 17 effects that the bulges caused by the measuring head 14 and the connecting wires 15 form substantially in the direction of the pressure chamber 17. For the contact to the wall of the trachea 3 a substantially unhindered envelope sealing surface 5 is produced, i.e. the outer side of the outer balloon 7, so that caused by the arrangement of the temperature sensor 13 on the cuff 4 an additional traumatic stress of the trachea is not produced.

The arrangement of the temperature sensor 13 between the inner balloon 6 and the outer balloon 7 of the cuff 4 first of all enables a safe build-up of the inflation pressure in the unobstructed pressure space 17 between the inner balloon 6 and the breathing tube 2 and a comfortable rest of the measuring head 14 or the overlying protective foil 18 on the inner wall of the outer balloon 7. Since the outer balloon 7 of the cuff 4 caused by the inflation pressure in the pressure chamber 17 rests tightly on the wall of the trachea 3, a tight contact of the measuring head 14 to the wall of the trachea 3 is obtained. The resistance to be bridged for temperature measurement is only limited to the small wall thickness of the outer balloon 7. Additional negative parameters and disturbances resulting from particularly inaccurate distances of the measuring head 14 to the wall of the trachea 3, can thereby be eliminated. The arrangement of a thermally conductive protective foil 18, e.g. a self-adhesive aluminum foil above the measuring head 14 further improves the measurement of the body core temperature, since possible unevenness caused by the shape of the measuring head, the attachment on the inner balloon 6 and the connection of the connecting wires 15 on the measuring head 14 are compensated or bypassed. Through the head conductance of the protective foil, a larger surface area of the trachea can be utilized for determining the body temperature.

After positioning the tracheal tube 1 in the trachea 3 of the patient and after sealing the breathing tube 2 against the trachea 3 by means of the trachea sealing cuff 4, respiratory gas or breathing air flows during inspiration from a pressure or volume controlled respirator via the breathing tube 2 into the lungs of the patient, according to the arrows shown in Fig. 1. Usually, the entire breathing air generated by the breathing apparatus flows into the patient's lungs. During expiration, the pressure and the volume of the breathing air first of all reduces so that caused by the missing pressure or volume of the breathing apparatus the breathing air flows back from the lungs of the patient through the breathing tube 2. Since the cuff 4 independent of the inspiration or expiration by the breathing apparatus permanently contacts the trachea 3 via the envelope sealing surface 5, since the gas volume in the pressure chamber 17 is permanently pressurized and is held by the return function of the connecting piece 12, a safe measurement of the body core temperature is possible independent of the respiration cycle.

## Claims

1. Tracheal tube (1) for ventilating patients by using a breathing tube (2), which is at least partially insertable into the trachea (3), comprising a cuff (4) arranged at the part insertable into the trachea (3) around the breathing tube (2), and comprising a temperature sensor (13),
**characterized in that** the cuff (4) comprises an outer balloon (7) and an inner balloon (6) and the temperature sensor (13) is attached on the outer side of the inner balloon (6).

2. Tracheal tube (1) as claimed in claim 1,
**characterized in that** the temperature sensor (13) is attached on the outer side of the inner balloon (6) by means of an adhesive.

3. Tracheal tube (1) as claimed in claim 1,
**characterized in that** the temperature sensor (13) is embedded into the outer side of the inner balloon (6).

4. Tracheal tube (1) as claimed in one of claims 1 to 3,
**characterized in that** a protective layer (18) is provided and the protective layer (18) covers the temperature sensor (13) with respect to the outer balloon (7).

5. Tracheal tube (1) as claimed in one of claims 1 to 4,
**characterized in that** a protective layer (18) is provided and the protective layer (18) covers the temperature sensor (13) with respect to the inner balloon (6).

6. Tracheal tube (1) as claimed in claim 4 or 5,
**characterized in that** the protective layer (18) is a metal foil, preferably an aluminum foil.

7. Tracheal tube (1) as claimed in one of claims 1 to 6,
**characterized in that** the outer balloon (7) and the inner balloon (6) of the cuff (4) are made of the same material.

8. Tracheal tube (1) as claimed in one of claims 1 to 6,
**characterized in that** the inner balloon (6) is made of a more flexible material than the outer balloon (7).

9. Tracheal tube (1) as claimed in one of claims 1 to 8,
**characterized in that** the temperature sensor (13) is a NTC sensor.

10. Tracheal tube (1) as claimed in one of claims 1 to 9,
**characterized in that** the connecting wires (15) of the temperature sensor (13) extend up to the breathing tube (2) between the inner balloon (6) and the outer balloon (7).

11. Tracheal tube (1) as claimed in one of claims 1 to 10,
**characterized in that** the connecting wires (15) of the temperature sensor (13) are arranged at least in the part insertable into the trachea (3) within and/or integrated in the breathing tube (2).

## Patentansprüche

1. Trachealtubus (1) zur Beatmung von Patienten mit einem Beatmungsschlauch (2), der zumindest teilweise in die Trachea (3) einführbar ist, mit einer Dichtungsmanschette (4), die an dem in die Trachea (3) einführbaren Teil um den Beatmungsschlauch (2) herum angeordnet ist, und mit einem Temperatursensor (13),
**dadurch gekennzeichnet, dass** die Dichtungsmanschette (4) einen Außenballon (7) und einen Innenballon (6) aufweist und der Temperatursensor (13) auf der Außenseite des Innenballons (6) befestigt ist.

2. Trachealtubus (1) nach Anspruch 1,
**dadurch gekennzeichnet, dass** der Temperatursensor (13) auf der Außenseite des Innenballons (6) mittels eines Klebemittels befestigt ist.

3. Trachealtubus (1) nach Anspruch 1,
**dadurch gekennzeichnet, dass** der Temperatursensor (13) in der Außenseite des Innenballons (6) eingebettet ist.

4. Trachealtubus (1) nach einem der Ansprüche 1 bis 3,
**dadurch gekennzeichnet, dass** eine Schutzschicht (18) vorgesehen ist und die Schutzschicht (18) den Temperatursensor (13) zum Außenballon (7) hin abdeckt.

5. Trachealtubus (1) nach einem der Ansprüche 1 bis 4,
**dadurch gekennzeichnet, dass** eine Schutzschicht (18) vorgesehen ist und die Schutzschicht (18) den Temperatursensor (13) zum Innenballon (6) hin abdeckt.

6. Trachealtubus (1) nach Anspruch 4 oder 5,
**dadurch gekennzeichnet, dass** die Schutzschicht (18) eine Metallfolie, bevorzugt eine Aluminiumfolie ist.

7. Trachealtubus (1) nach einem der Ansprüche 1 bis 6,
**dadurch gekennzeichnet, dass** der Außenballon (7) und der Innenballon (6) der Dichtungsmanschette (4) aus dem gleichen Material hergestellt sind.

8. Trachealtubus (1) nach einem der Ansprüche 1 bis 6,
**dadurch gekennzeichnet, dass** der Innenballon (6) aus einem dehnfähigeren Material hergestellt ist als der Außenballon (7).

9. Trachealtubus (1) nach einem der Ansprüche 1 bis 8,
**dadurch gekennzeichnet, dass** der Temperatursensor (13) ein NTC-Sensor ist.

10. Trachealtubus (1) nach einem der Ansprüche 1 bis 9,
**dadurch gekennzeichnet, dass** die Anschlussdrähte (15) des Temperatursensors (13) bis zum Beatmungsschlauch (2) zwischen dem Innenballon (6) und dem Außenballon (7) verlaufen.

11. Trachealtubus (1) nach einem der Ansprüche 1 bis 10,
**dadurch gekennzeichnet, dass** die Anschlussdrähte (15) des Temperatursensors (13) zumindest in dem in die Trachea (3) einführbaren Teil innerhalb des und/oder integriert im Beatmungsschlauch (2) angeordnet sind.

## Revendications

1. Canule trachéale (1) pour ventiler les patients à l'aide d'un tube de respiration (2), que l'on peut introduire au moins partiellement dans la trachée (3), comprenant un manchon (4) disposé au niveau de la partie que l'on peut introduire dans la trachée (3) autour du tube de respiration (2), et comprenant un capteur de température (13),
**caractérisée en ce que** le manchon (4) comprend un ballon externe (7) et un ballon interne (6) et le capteur de température (13) est fixé sur le côté externe du ballon interne (6).

2. Canule trachéale (1) selon la revendication 1,
**caractérisée en ce que** le capteur de température (13) est fixé sur le côté externe du ballon interne (6) à l'aide d'un adhésif.

3. Canule trachéale (1) selon la revendication 1,
**caractérisée en ce que** le capteur de température (13) est incrusté dans le côté externe du ballon interne (6).

4. Canule trachéale (1) selon l'une des revendications 1 à 3,
**caractérisée en ce qu'**une couche de protection (18) est prévue et que la couche de protection (18) recouvre le capteur de température (13) par rapport au ballon externe (7).

5. Canule trachéale (1) selon l'une des revendications 1 à 4,
**caractérisée en ce qu'**une couche de protection (18) est prévue et que la couche de protection (18) recouvre le capteur de température (13) par rapport au ballon interne (6).

6. Canule trachéale (1) selon la revendication 4 ou 5,
**caractérisée en ce que** la couche de protection (18) est un film métallique, de préférence un film aluminium.

7. Canule trachéale (1) selon l'une des revendications 1 à 6,
**caractérisée en ce que** le ballon externe (7) et le ballon interne (6) du manchon (4) sont constitués du même matériau.

8. Canule trachéale (1) selon l'une des revendications 1 à 6,
**caractérisée en ce que** le ballon externe (6) est réalisé dans un matériau plus souple que le ballon externe (7).

9. Canule trachéale (1) selon l'une des revendications 1 à 8,
**caractérisée en ce que** le capteur de température (13) est un capteur NTC.

10. Canule trachéale (1) selon l'une des revendications 1 à 9,
**caractérisée en ce que** les fils de connexion (15) du capteur de température (13) s'étendent jusqu'au tube de respiration (2) entre le ballon interne (6) et le ballon externe (7).

11. Canule trachéale (1) selon l'une des revendications 1 à 10,
**caractérisée en ce que** les fils de connexion (15) du capteur de température (13) sont disposés dans au moins la partie que l'on peut introduire dans la trachée (3) dans et/ou sont intégrés dans le tube de respiration (2).
